(19) Europäisches Patentamt
European Patent Office
Office européen des brevets

(11) Numéro de publication : **0 157 842 B1**

# (12) FASCICULE DE BREVET EUROPÉEN

(45) Date de publication du fascicule du brevet : **19.07.89**

(51) Int. Cl.[4] : **C 07 J 7/00**, C 07 J 13/00

(21) Numéro de dépôt : **84903643.9**

(22) Date de dépôt : **04.10.84**

(86) Numéro de dépôt international : **PCT/FR 84/00219**

(87) Numéro de publication internationale : **WO/8501504 (11.04.85 Gazette 85/09)**

## (54) NOUVEAU PROCEDE DE PREPARATION DE DERIVES DE LA SERIE DE LA 17-g(a)-HYDROXY 19-NOR-PROGESTERONE.

(30) Priorité : **04.10.83 FR 8315759**

(43) Date de publication de la demande : **16.10.85 Bulletin 85/42**

(45) Mention de la délivrance du brevet : **19.07.89 Bulletin 89/29**

(84) Etats contractants désignés : **AT BE CH DE FR GB LI LU NL SE**

(56) Documents cités :
**FR-A- 1 453 221**
**FR-A- 2 271 833**
**Le dossier contient des informations techniques présentées postérieurement au dépôt de la demande et ne figurant pas dans le présent fascicule.**

(73) Titulaire : **THERAMEX S.A.**
**2, boulevard Charles III**
**Monte Carlo (MC)**

(72) Inventeur : **TCHERNATINSKY, Claude**
**Chemin de Revoirs 12 bis, avenue Victor Hugo**
**F-06240 Beausoleil (FR)**

(74) Mandataire : **Burtin, Jean-François**
**c/o GENIFAR 28, Rue du Val d'Or**
**F-92150 Suresnes (FR)**

**EP 0 157 842 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente demande concerne un nouveau procédé d'obtention de dérivés de la 17α-hydroxy 19-norprogestérone répondant à la formule générale I

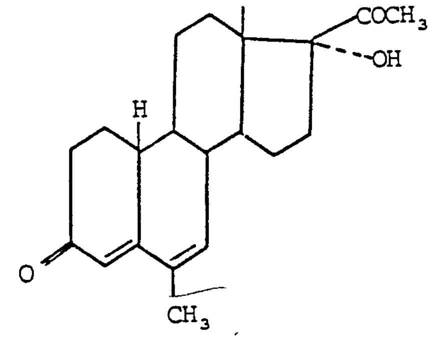

(I)

et leur conversion éventuelle en composé 17α-acylé, dérivé d'un acide carboxylique alphatique saturé ayant de 1 à 18 atomes de carbone en chaîne droite ou ramifiée ou insaturé ayant de 2 à 18 atomes de carbone et comportant de 1 à 5 doubles liaisons.

L'invention a plus particulièrement pour objet un nouveau procédé d'obtention des composés de formule générale I plus économique et plus aisément réalisable à l'échelle industrielle car il utilise des matières premières commercialement accessibles en grandes quantités.

En effet, les composés de formule générale I présentent un grand intérêt d'ordre technique et thérapeutique car ils manifestent des propriétés très intéressantes notamment en tant que médicament progestatif ou médicament anti-androgène.

Ces composés ont déjà été décrits dans la littérature et on peut trouver des informations sur leur mode de préparation et sur les propriétés de ces composés, en particulier dans le brevet britannique 1.515.284 (J.M. Gastaud). De même, la publication Arzneimittel Forschung 33 (1983) pp. 710 à 715 a fourni une étude détaillée des propriétés pharmacologiques du principal représentant de cette classe, la 17α-acetoxy 6-methyl 19-nor 4-6 pregnadiène 3,20-dione.

Dans le procédé antérieurement décrit, les composés de formule générale I étaient préparés au départ de la 17α-hydroxy 19-nor 4-pregnène 3,20-dione ou 19-nor progestérone, une substance stéroïdienne de la série du 19-nor prégnane.

La présente invention vise à réaliser cette synthèse au départ de composés appartenant à la série de l'estrane en l'occurrence le 3,17-dicéto estrène-4 ou le 3-méthoxy 17-céto estra 1,3,5(10)-triène, substances aisément accessibles et auxquelles il a été possible de faire subir des transformations chimiques simples, connues de l'homme de l'art pour d'autres molécules analogues.

Le procédé comprend les étapes dans lesquelles on soumet un 3-alcoxy 19-nor pregna 3,5(10),17(20)-triène de formule générale II

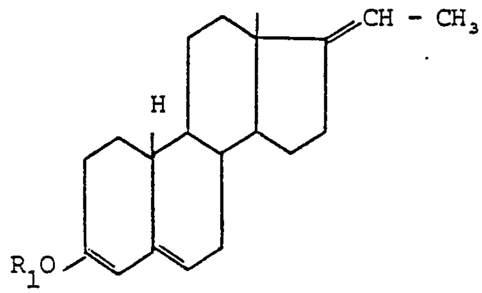

(II)

(sous forme d'isomère E ou Z) dans laquelle $R_1$ est un radical alcoyle inférieur ou cycloalcoyle inférieur à l'action d'un réactif formylant dans les conditions de la réaction de Vilsmeir-Hack pour obtenir un dérivé 6-formylé de formule générale III

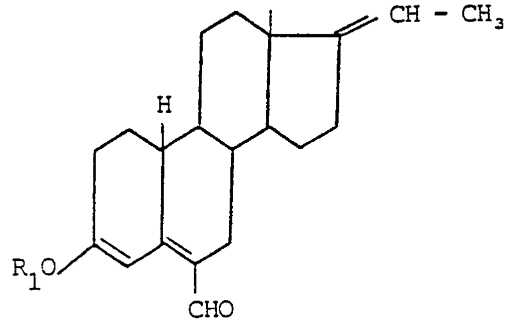

(III)

(sous forme d'isomère E ou Z) dans laquelle $R_1$ est un radical alcoyle inférieur ou cycloalcoyle inférieur que l'on réduit par action d'un hydrure mixte de métal alcalin en dérivé 6-hydroxyméthylé de formule générale IV

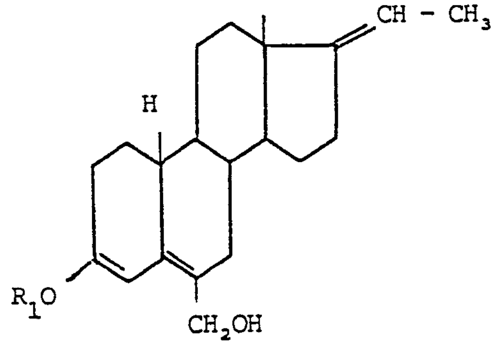

(IV)

(sous forme d'isomère E ou Z) dans laquelle $R_1$ est défini comme précédemment soumet celui-ci à l'action d'un acide fort ou d'un acide organique pour obtenir le dérivé 3-céto 6-méthylénique de formule V

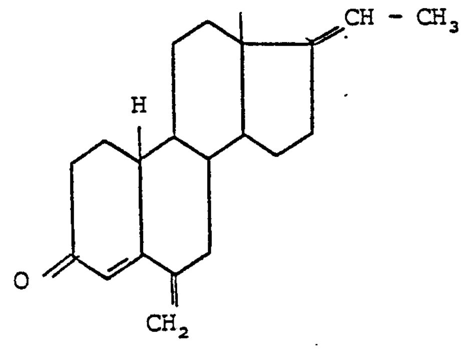

(V)

(sous forme d'isomère E ou Z) isomérise celui-ci par réaction avec un catalyseur d'isomérisation pour obtenir un dérivé 3-céto 19-nor pregna 4,6,17(20)-triénique de formule VI

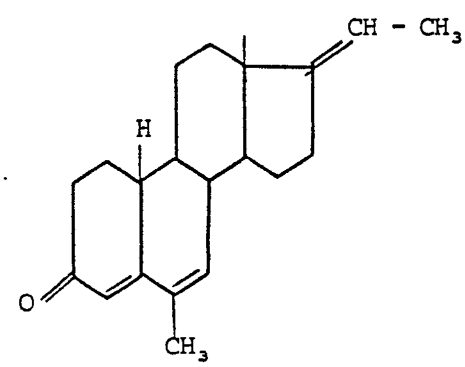

(VI)

(sous forme d'isomère E ou Z) et caractérisé en ce que par action d'un réactif de bis-hydroxylation, formé par emploi simultané d'une quantité de tétroxyde d'osmium combinée à une quantité molaire importante d'un hydroperoxyde de N-oxyde d'amine tertiaire pour obtenir le 17α-hydroxy 19-nor pregna 4,6-diène de formule I

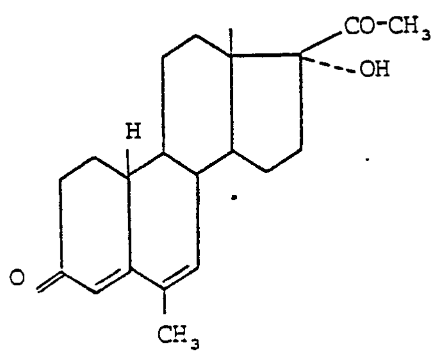

(I)

lequel, le cas échéant par acylation au moyen d'un dérivé fonctionnel d'acide organique carboxylique, fournit le dérivé 17α acyloxylé de formule générale

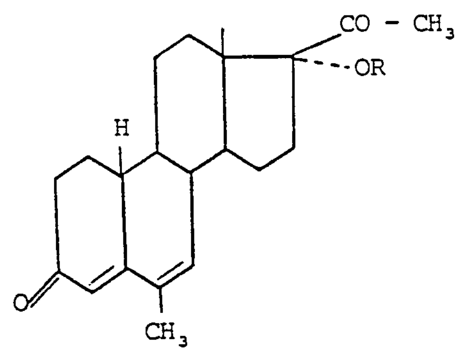

dans laquelle R représente le reste acyle d'un acide organique carboxylique saturé ou non saturé tel que défini précédemment.

L'invention est définie comme suit :

1) L'oxydation du composé de formule VI est effectuée à l'aide d'un réactif de bis-hydroxylation tel que, par exemple, l'hydroperoxyde de N-oxyde de triéthylamine ou l'hydroperoxyde de N-oxyde de morpholine.

2) La réaction est conduite au sein d'un solvant inerte comme par exemple un alcool tertiaire, le terbutanol étant le solvant préféré.

3) La réaction d'oxydation est conduite simultanément en présence de quantités catalytiques de tétroxyde d'osmium et d'une base organique telle que, par exemple, la pyridine.

L'invention comprend également les composés intermédiaires obtenus au cours du procédé, objet de la présente invention, à savoir :

— les dérivés 6-formylés de formule générale III et notamment les 3-méthoxy 6-formyl 19-nor pregna 3,5,17(20)-triène

— les dérivés 6-hydroxyméthylés de formule générale IV et notament le 3-méthoxy 6-hydroxyméthyl 19-nor pregna 3,5,17(20)-triène

— les dérivés 6-méthyléniques de formule générale V et notamment les 3-céto 6-méthylène 19-nor pregna 4,17(20)-diène

— les dérivés 6-méthyl pregnatriéniques de formule VI et notamment les 3-céto 6-méthyl 19-nor pregna 4,6,17(20)-triène.

Le procédé de la présente invention se différencie de l'art antérieur et notamment du procédé général décrit dans le brevet français 1 453 221 et ce, pour deux raisons précises :

a) le réactif utilisé dans le brevet français 1 453 221 n'est pas le même que celui utilisé dans la présente demande

b) la structure chimique à laquelle est appliquée le procédé de synthèse n'est pas la même que celles envisagées dans le brevet 1 453 221 (ROUSSEL-UCLAF)

En effet, l'analyse du brevet 1 453 221 montre que la réaction utilisée pour la bis-hydroxylation et le réactif utilisé pour cette réaction sont sensiblement distincts de celui que nous utilisons dans le procédé revendiqué.

Si on se réfère à l'exemple le plus détaillé du brevet français 1 453 221, on constate au stade B l'emploi successif d'une quantité importante d'un réactif à base de tétroxyde d'osmium avec mise en contact, puis de l'hydroperoxyde de N-oxyde d'amine tertiaire.

Le rendement en produit oxydé est faible (0.66 g pour 1,344 g de produit de départ).

Au contraire, dans le procédé qui se trouve revendiqué dans la présente demande, on utilise simultanément une quantité catalytique de tétroxyde d'osmium et une quantité molaire importante d'hydroperoxyde de N-oxyde d'amine tertiaire.

Selon les modalités particulières actuellement préférées, le procédé peut encore être explicité comme suit :

a) Le 3-alcoxy 19-nor pregna 3,5,17(20)-triène (isomère E ou Z) est formylé en position 6 par un réactif de VILSMEIER, obtenu notamment par action du phosgène ou d'oxychlorure de phosphore sur un formamide substitué comme le diméthyl formamide, la réaction pouvant être conduite au sein du même solvant avec ou sans adjonction d'un tiers solvant agissant comme diluant. Dans ce cas, les solvants chlorés sont les solvants préférés. On obtient ainsi un 3-alcoxy 6-formyl 19-nor pregna 3,5,17(20)-triène, sous forme d'isomère E ou Z, de formule générale III.

b) Le composé formylé de formule générale IV est réduit par un réactif réducteur comme par exemple un hydrure mixte de métal alcalin et particulièrement un borohydrure de métal alcalin (sodium, potassium ou lithium).

Cette transformation fournit un 3-alcoxy-6-hydroxyméthyl 19-nor pregna 3,5,17(20)-triène, sous forme d'isomère E ou Z, répondant à la formule générale IV.

c) Le dérivé 6-hydroxyméthylé est déshydraté par réaction au sein d'un solvant inerte avec un acide minéral fort, comme l'acide chlorhydrique ou l'acide sulfurique, ou avec un acide organique comme l'acide oxalique ou l'acide acétique, pour obtenir le composé V.

d) L'isomérisation du composé V en 3-céto 6-méthyl pregna-4,6,17(20)-triène (IV) s'effectue sous l'influence d'un catalyseur d'isomérisation comme, par exemple, le charbon palladié au sein d'un solvant inerte, un alcanol par exemple à des températures pouvant aller de la température ambiante à une température de l'ordre de 120°. Il est en outre possible d'ajouter au milieu réactionnel une ou des substances susceptibles de jouer un rôle de substance tampon comme par exemple, l'acétate de sodium ou l'acétate de potassium.

e) L'oxydation du composé VI en dérivé I 17α-hydroxylé est effectuée comme décrit précédemment.

f) Le dérivé 17α-hydroxylé résultant est éventuellement acylé par un dérivé fonctionnel d'acide organique carboxylique comme par exemple un halogénure d'acide, un anhydride d'acide, un anhydride mixte ou encore l'anhydride mixte formé par réaction d'un acide organique avec un dialcoyl — ou dicycloalcoyl carbo di-imide pour obtenir un dérivé 17α-acyloxylé dans lequel R est le reste acylé d'un acide organique carboxylique aliphatique saturé ayant de 1 à 18 atomes de carbone en chaîne droite ou ramifiée, ou insaturé ayant 2 à 18 atomes de carbone et comportant de 1 à 5 doubles liaisons.

Dans les formules précédentes, les produits sont soit des isomères E ou Z purs ou un mélange en proportions quelconques de ces deux formes.

Les exemples suivants illustrent l'invention, ils ne la limitent en aucune façon.

Préparation des matières premières de formule générale II

A) 3-méthoxy 19-nor pregna 3,5,17(20)-triène (isomère E)

a) à partir de 17-ethinyl-estradiol 3-methyl-ether

En utilisant la méthode décrite par F. B. Colton J. of Am. Chem. Soc. 79, 1123 (1957) au départ du 17α-éthinyl estradiol 3-méthyl éther, on obtient la 19-nor pregna 4,17(20)-diène 3-one qui, par action de l'orthoformiate de méthyle en milieu acide, conduit au 3-méthoxy 19-nor pregna-3,5,17(20)-triène (isomère E).

b) à partir du 3-méthoxy 19-nor pregna 2,5,10 à 17(20)-trene (isomère E)

On dissout 21 g de 3-méthoxy 19-nor pregna 2,5(10)17(20)-triène (isomère E) dans 420 ml de chloroforme. On y ajoute 0,21 g de chlorure de tris (triphényl-phosphine) rhodium et on porte le mélange au reflux pendant une heure. On concentre ensuite sous vide et laisse cristalliser le résidu sec après reprise dans le méthanol. On obtient ainsi 17 g de 3-méthoxy 19-nor pregna-3,5,17(20)-triène (isomère E), soit un rendement de 81 %. Le produit fond à 94 °C.

c) à partir de 3,17-dioxo estra-4-ène

a) 3-méthoxy 17-oxo estra-3,5 diène

10 g de 3,17-dioxo estra 4-ène sont agités 1 heure dans 50 ml de méthanol avec 10 ml d'orthoformiate de méthyle et 50 mg d'acide p. toluène sulfonique. On ajoute quelques gouttes de triéthylamine, puis on glace et essore. On obtient 69 % de 3-méthoxy 17-oxo estra-3,5 diène.

b) On mélange sous agitation 90 ml de tétrahydrofuran anhydre, 9 g de (cyclohexylimino)-2 éthyl phosphonate de diéthyle et 1 g d'hydrure de sodium en suspension huileuse à 80 % sous azote, à 0 °C pendant 1 heure. On y ajoute 7 g de 3-méthoxy 17-oxo estra-3,5 diène et on maintient sous agitation à la température ambiante pendant 24 heures. On ajoute lentement une solution de 30 ml d'eau, 7 ml d'acide acétique et 14 g d'acétate de potassium et agite 2 heures, puis on additionne d'une solution saturée de chlorure de sodium. On décante les phases organiques et après les extractions et lavages habituels avec une solution saturée de chlorure de sodium, on évapore à sec le solvant et on obtient le 3-méthoxy-21-oxo 19-nor pregnatriène 3,5,17(20) brut. Ce produit, dissous dans 45 ml de tétrahydrofuranne est traité 1 heure à reflux avec une solution éthérée de 2 g d'hydrure de lithium aluminium et 4 g de chlorure d'aluminium anhydre. Le milieu est refroidi, traité par une solution aqueuse de chlorure d'ammonium et après extraction on isole 5,5 g de 3-méthoxy 19-nor pregna 3,5,17(20)-triène isomère E.

B) 3-éthoxy-19-nor pregna 3,5,17(20)-triène (isomère E)

5 g de 3-oxo 19-nor pregna 4,17(20)-diène (isomère trans) sont dissous dans 50 ml d'éthanol. On ajoute 5 ml d'orthoformiate d'éthyle et 25 mg d'acide p. toluène sulfonique. On agite pendant 15 mn à température ambiante, puis on neutralise avec quelques gouttes de tri éthylamine. On concentre à faible volume puis on glace et laisse cristalliser en glacière. On sépare les cristaux par filtration et on les essore. On obtient ainsi le 3-éthoxy 19-nor pregna 3,5,17(20)-triène (isomère E avec un rendement de 78 %.

C) 3-méthoxy-19-nor pregna 3,5,17(20)-triène (isomère Z).

a) à partir de 3-méthoxy 17-oxo estra 1,3,5(10) triène.

Ce produit est converti en 3-méthoxy 19-nor pregna 1,3,5(10),17(20)-tétraène (isomère Z) selon la méthode décrite par Krubiner et Oliveto J. Org. Chem. 31 (1966) 24. Celui-ci est réduit selon la méthode de Birch-Nelson pour fournir après recristallisation de l'éthanol, 79 % de 3-méthoxy 19-nor pregna 2,5(10)17(20)-triène (isomère cis) fondant à 113°.

Par action de l'acide chlorhydrique dilué on forme ensuite le 3-oxo 19-nor pregna 4,17(20)-diène (isomère Z). Ce dernier est converti en 3-méthoxy-19-nor pregna 3,5,17(20)-triène (isomère Z) par action de l'orthoformiate de méthyle en présence d'acide p. toluène sulfonique. De la même façon on forme le 3-éthoxy 19-nor pregna 3,5,17(20)-triène isomère Z (F = 125° [α] D = — 181°. λmax 242 mμ ε = 19160).

b) à partir de 3-méthoxy-19-nor pregna 2,5,(10)17(20)-triène (isomère Z)

On effectue l'isomérisation selon le mode opératoire décrit au paragraphe A b).

c) à partir du 3,17-dioxo estra 4-ène

On dissout 9,6 g de 3-méthoxy 17-oxo estra 3,5-diène dans 30 ml de diméthylsulfoxyde et on ajoute 7 g de bromure de (triphényléthyl) phosphonium, puis 2 g de terbutylate de potassium. On chauffe le mélange 16 heures à 60°, puis après refroidissement on extrait à l'hexane. Les phases hexaniques sont amenées à sec, puis le résidu sec est repris par du méthanol d'où il cristallise. On obtient ainsi le 3-méthoxy-19-nor pregna-3,5,17(20)-triène (isomère Z) avec un rendement de 84 %.

On prépare de la même façon le 3-éthoxy-19-nor pregna 3,5,17(20)-triène (isomère Z) à partir du 3-éthoxy 19-oxo estra 3,5-diène
(F = 125°, [α] D = — 181°, λmax = 242 mμ ε = 19160).

Exemple I

3,20 dioxo 6-méthyl 17α-hydroxy 19-nor pregna 4,6-diène

Stade A : 3-méthoxy 6-formyl 19-nor pregna-3,5,17(20)-triène (isomère E)

A une solution de 16,4 g de 3-méthoxy 19-nor pregna-3,5,17(20)-triène (isomère E) dans 135 ml de diméthylformamide on ajoute le réactif de Vilsmeier formé de diméthylformamide (72 ml), oxychlorure de phosphore (9 ml) à 0 °C. Après 1/2 heure de contact on ajoute lentement 33 g d'acétate de sodium dans 80 ml d'eau, on extrait la phase aqueuse par du chlorure de méthylène, qu'on lave par une solution de bicarbonate de sodium. Après traitements habituels le résidu est cristallisé dans le méthanol pour obtenir le 3-méthoxy 6-formyl 19-nor pregna 3,5,17(20)-triène (isomère E) avec 84 % de rendement.
F = 110°, spectre UV max 323 nm ε = 12900

De la même manière, on obtient le 3-éthoxy 6-formyl 19-nor pregna 3,5,17(20)-triène (isomère Z) à partir du 3-éthoxy 19-nor pregna 3,5,17(20)-triène (isomère Z).
(F = 110° [α] D = — 240 λmax 324 nm ε = 14400).

Stade B : 3-methoxy 6-hydroxyméthyl 19-nor pregna 3,5,17(20)-triène (isomère E).

12 g de 3-méthoxy 6-formyl 19-nor pregna 3,5,17(20)-triène (isomère trans) dissous dans 120 ml de méthanol et 720 mg de borohydrure de sodium sont agités 45 mn à 5 °C, puis on ajoute 80 ml d'eau, essore et sèche les cristaux de 3-méthoxy 6-hydroxyméthyl 19-nor pregna-3,5,17(20)-triène (isomère E) (95 % de rendement). De la même manière on obtient le 3-méthoxy 6-hydroxyméthyl 19-nor pregna 3,5,17(20)-triène (isomère Z) à partir du 3-méthoxy 6-formyl 19-nor pregna 3,5,17(20)-triène (isomère Z).

Stade C : 3-oxo 6-méthylène 19-nor pregna 4,17(20)-diène (isomère E).

10 g de 3-méthoxy-6 hydroxy méthyl 19-nor pregna 3,5,17(20)-triène (isomère E) dans 90 ml de méthanol et 10 ml d'acide chlorhydrique 2N sont agités 5 mn, puis on essore, lave et sèche les cristaux de 3-oxo 6-méthylène 19-nor pregna 4,17(20)-diène (isomère E) rendement : 97 % (F = 105° λmax 264 nm ε = 10500). De la même manière on obtient le 3-céto 6-méthylène 19-nor pregna 4,17(20)-diène (isomère Cis) à partir de 3-méthoxy 6-(hydroxyméthyl) 19-nor pregna 3,5,17(20)-triène (isomère Cis).
(F = 148° [α] D = + 206 λmax 266 nm ε = 11500

Stade D : 3-oxo 6-méthyl 19-nor pregna 4,6,17(20)-triène (isomère trans)

2 g de 3-oxo 6-méthylène 19-nor pregna 4,17(20)-triène (isomère E), 2 g d'acétate de sodium 800 mg de charbon palladié à 5 % de palladium dans 200 ml d'éthanol à 95 %, sont portés à reflux 1 heure, puis on filtre et amène à sec. On reprend par du chloroforme, lave par de l'eau et amène à sec à nouveau. On

obtient un produit huileux après distillation, constitué principalement de 3-oxo 6-méthyl 19-nor pregna 4,6,17(20)-triène (isomère E).

[α] D = — 35° λmax 290 nm ε = 22100.

De la même manière, on obtient le 3-oxo 6-méthyl 19-nor pregna 4,6,17(20)-triène (isomère Z)n à partir de 3-céto 6-méthylène 19-nor pregna 4,17(20)-diène (isomère Z), sous forme d'un produit huileux [α] D = + 92°.

Exemple II

10 g de 3-oxo 6-méthyl 19-nor pregna 4,6,17(20)-triène (isomère Z), 100 ml de ter butanol et 50 mg de tétroxyde d'osmium. Ajouter 14 g de complexe triéthylamine-oxyde, hydroperoxyde, et laisser agiter 24 heures à température ambiante. Ajouter 10 g de celite, 6 g de $SO_3Na_2$ dans 200 ml d'eau, extraire par le toluène et filtrer la solution toluénique sur une couche de silice pour chromatographie. L'éluat est amené à sec, et cristallisé dans le méthanol pour donner 63 % de 3,20-dicéto 6-méthyl 17α-hydroxy 19-nor pregna 4,6-diène

F = 206° λmax 201 nm ε = 24000.

Le même produit est obtenu par la même technique à partir de 3-oxo 6-méthyl 19-nor pregna 4,6,17(20)-triène (série trans).

Exemple III

10 g de 3,20 dicéto 6-méthyl 17α-hydroxy 19-nor pregna 4,6-diène sont dissous dans 50 ml de chloroforme. 7 ml d'anhydride acétique et 1 g d'acide para toluène sulfonique sont ajoutés à cette solution.

Le mélange est porté au reflux sous azote pendant 1 heure, puis refroidi vers 40 °C, et additionné de 10 ml de méthanol et de 1 ml d'acide chlorhydrique concentré. Porter à nouveau au reflux pendant 1 heure, refroidir à température ambiante, laver par de la soude diluée puis par de l'eau jusqu'à neutralité. On distille à sec les phases organiques. Le produit repris par le méthanol laisse déposer des cristaux de 3,20 dicéto 6-méthyl 17α-acétoxy 19-nor pregna 4,6-diène. Le rendement est de 80 %.

**Revendications**

1. Un procédé d'obtention du 17α-hydroxy 6-méthyl 19-nor pregna 4,6-diène 3,20-dione de formule générale I

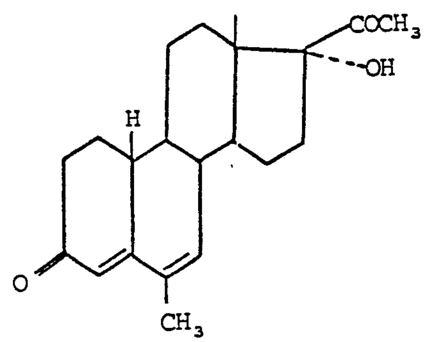

(I)

et conversion éventuelle en composé 17-acylé, dérivé d'un acide organique carboxylique aliphatique saturé ayant de 1 à 18 atomes de carbone en chaîne droite ou ramifiée, ou insaturé ayant de 2 à 18 atomes de carbone et comportant de 1 à 5 doubles liaisons, dans lequel on soumet un 3-alcoxy 19-nor pregna 3,5(10),17(20)-triène II à l'action d'un réactif formylant dans les conditions de la réaction de VILSMEIER-HACK, pour obtenir le dérivé 6-formylé de formule générale III

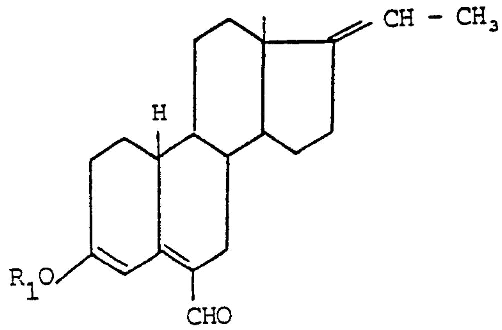

(III)

(sous forme d'isomère E ou Z)

dans laquelle $R_1$ est un radical alcoyle inférieur ou cycloalcoyle inférieur que l'on réduit par action d'un hydrure mixte de métal alcalin en dérivé 6-hydroxyméthylé de formule générale IV

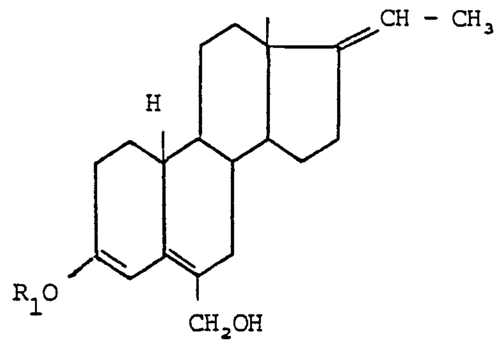

(IV)

(sous forme d'isomère E ou Z) dans laquelle $R_1$ est défini comme précédemment soumet celui-ci à l'action d'un acide fort, ou d'un acide organique pour obtenir le dérivé 3-céto-6 méthylénique de formule générale V

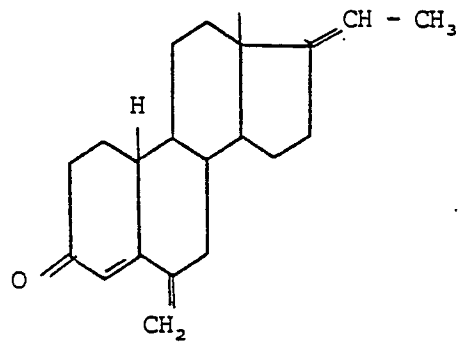

(V)

(sous forme d'isomère E ou Z) et isomérise celui-ci par réaction avec un catalyseur d'isomérisation pour obtenir le 3-céto 6-méthyl 19-nor 4,6,17(20)-pregnatriène (VI) caractérisé en ce que l'on soumet ce composé 3-céto 19-nor Δ 4,6,17(20)-pregnatriénique de formule générale VI

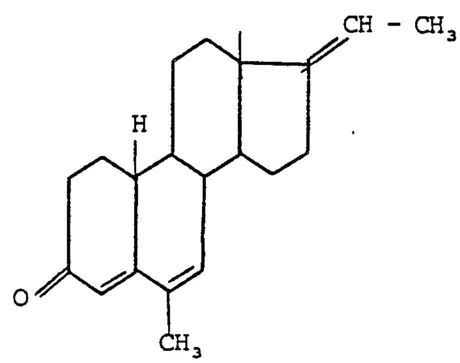

(VI)

(sous forme d'isomère E ou Z) à l'action d'un réactif de bis-hydroxylation forme par emploi simultané d'une quantité de tétroxyde d'osmium combinée à une quantité molaire importante d'un hydroperoxyde de N-oxyde d'amine tertiaire pour obtenir le 17α-hydroxy 19-nor Δ 4,6-pregnadiène de formule générale I

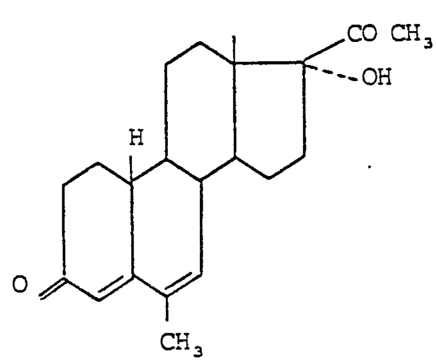

(I)

et si désiré transforme celui-ci par acylation au moyen d'un dérivé fonctionnel d'acide organique carboxylique en dérivé 17α-acylé.

2. Un procédé selon la revendication 1 dans lequel la réaction de bis-hydroxylation est conduite dans un alcool tertiaire inerte en présence d'une base pyridique.

3. A titre de produits intermédiaires les 19-nor pregnatriènes de formule générale III

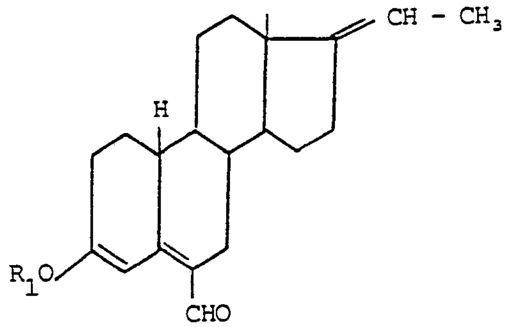

(III)

(sous forme d'isomère E ou Z) dans laquelle $R_1$ est un radical alcoyle inférieur ou cycloalcoyle inférieur.

4. A titre de produits intermédiaires les dérivés 6-hydroxyméthylés de formule générale IV

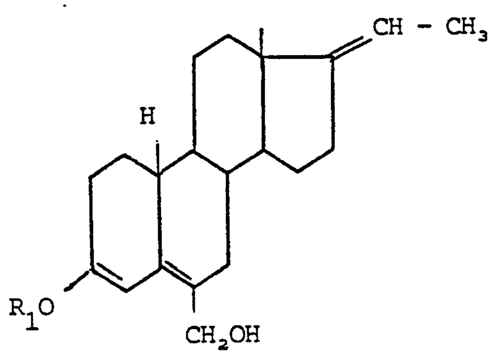

(IV)

(sous forme d'isomère E ou Z) dans laquelle $R_1$ est défini comme à la revendication 3.

5. A titre de produit intermédiaire le dérivé 3-céto 6-méthylénique de formule V

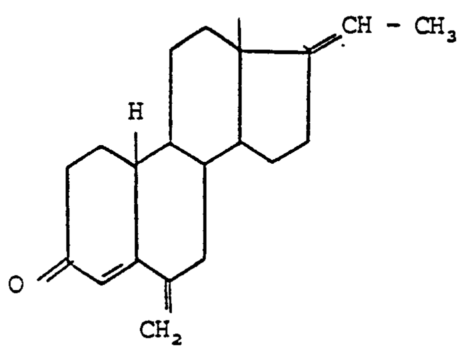

(V)

(sous forme d'isomère E ou Z).

6. A titre de produit intermédiaire le 3-céto 19-nor pregna 4,6,17(20)-triène de formule générale VI

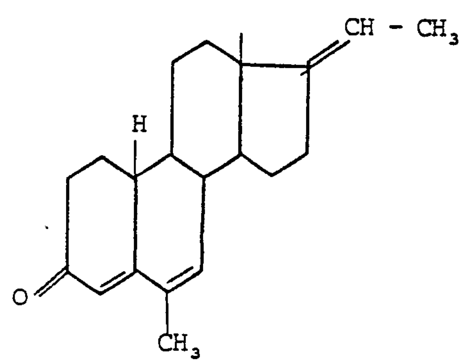

(VI)

(sous forme d'isomère E ou Z).

**Claims**

1. A process for producing 17α-hydroxy 6-méthyl 19-nor pregna 4,6-dien 3,20-dione of general formula I

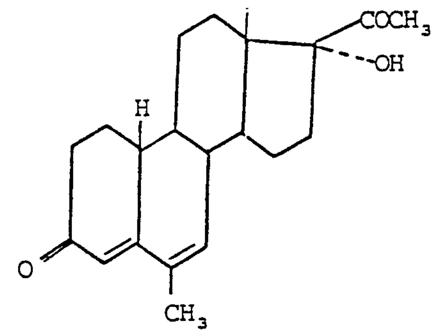

(I)

and optional conversion into a 17-acyloxylated derivative from a saturated a liphatic organic carboxylic acid having from 1 to 18 carbon atoms in straight or branched chain, or an unsaturated organic carboxylic acid having from 2 to 18 carbon atoms and including from 1 to 5 double bonds in wich a 3-alkoxy 19-nor pregna 3,5(10)-17(20)-Trien of formula II is submitted to the action of a formylating reagent under the conditions of the Vilsmeier-Hack's reaction to produce a 6-formylated derivative of general formula III

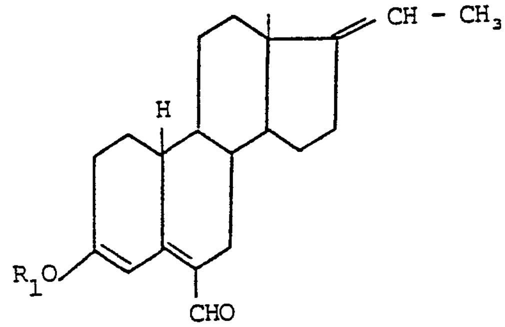

(III)

(in the form of an E or Z isomer) in which $R_1$ is a lower alkyl radical or a lower cycloalkyl radical wich is reduced by means of an alkali metal mixed hydride into a 6-hydroxymethylated derivative of general formula IV

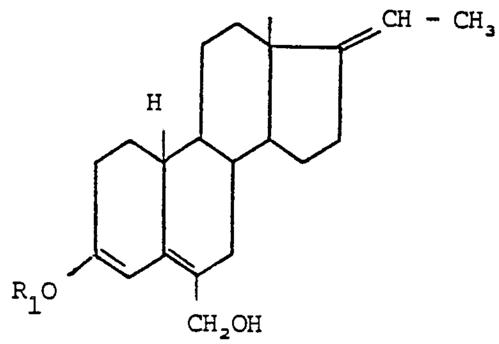

(IV)

(in the form of an E or Z isomer) wherein $R_1$ is defined as previously-given the 6-hydroxy methylated derivative is submitted to the action of a strong acid or an organic to produce a 3-keto 6-methylenic derivative of general formula V

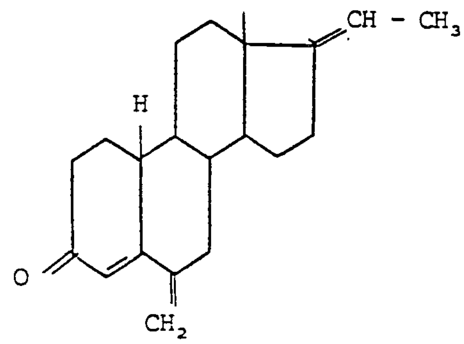

(V)

(in the form of an E or Z isomer) and the latter is isomerized by reacting it with an isomerizing catalyst to get the 3-keto 6-methyl 19-nor 4,6,17(20) pregna trien of formula VI characterized in that this 3-keto 19-nor Δ4,6,17(20) pregnatrienic compound of general formula VI

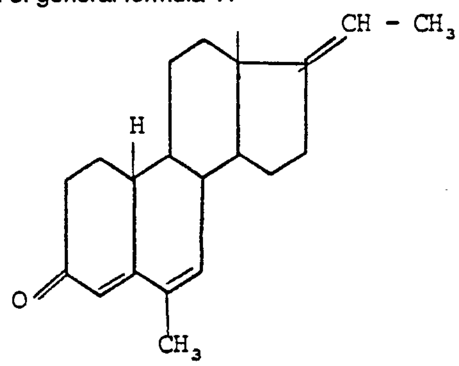

(VI)

(in the form of an E or Z isomer) is submitted to the action of a bishydroxylating reagent made of simultaneous utilization of an amount of Osmium Tetroxyde combined to a significant molar amount of a hydroperoxyde of a N-oxyde of a tertiary amine, to produce 17α-hydroxy 19-nor Δ-4,6-pregnadien of general formula I

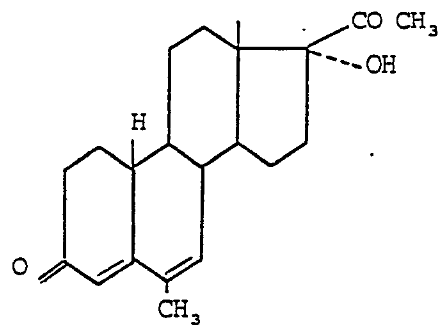

(I)

and which, when desired, is converted by acylation by means of a functional derivative of an organic carboxylic acid, into a 17α-acylated derivative.

2. A process according to claim 1 in which the bis hydroxylation reaction is performed into an inert tertiary alcohol in the presence of a pyridic base.

3. As intermediate compounds, the 19-nor pregnatriens of general formula III

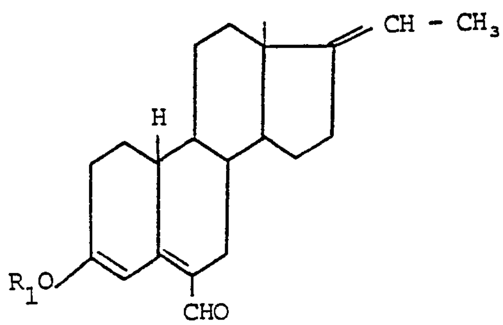

(III)

(in the form of an E or Z isomer) wherein $R_1$ is a lower alkyl or a lower cycloalkyl radical.

4. As intermediate products, the 6-hydroxymethylated derivatives of general formula IV

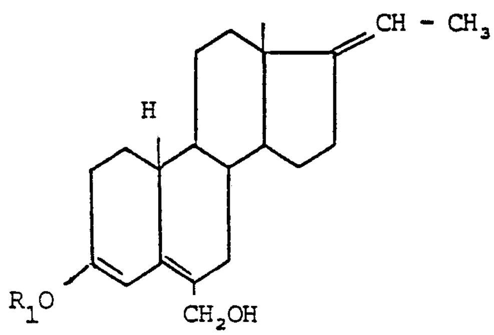

(IV)

(in the form of an E or Z isomer) wherein $R_1$ is defined as in claim 3.

5. As intermediate compound the 3-keto 6-methylenic derivative of formula V

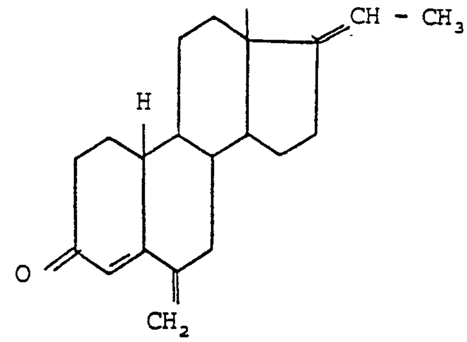

(V)

(in the form of an E or Z isomer).

6. As intermediate compound, 3-keto 19-nor pregna 4,6,17(20)-Trien of general formula VI

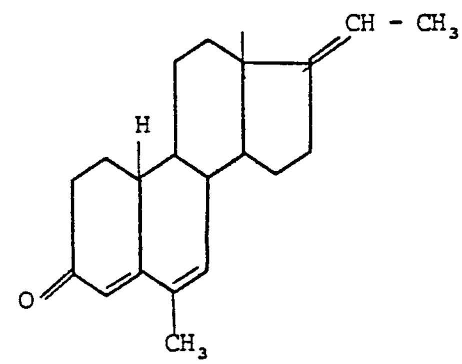

(VI)

(in the form of an E or Z isomer).

## Patentansprüche

1. Verfahren zur Herstellung von 17α-Hydroxy 6-methyl 19-nor pregna 4,6-dien 3,20-dion der allgemeinen Formeln I

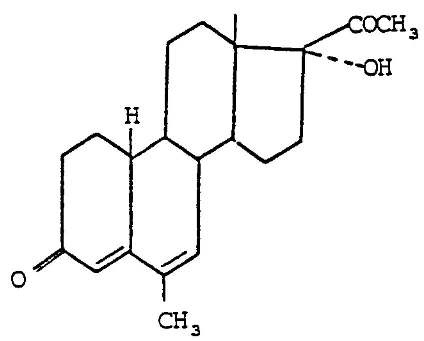

(I)

und gegebenenfalls Umsetzung dieses Produkts, in einer 17-acylierten Derivate einer gësattigten gerade- oder verzweigtkettigen aliphatischen organischen Carbonsäure mit 1 bis 18 Kohlenstoffatomen, oder einer ein-bis fünffach un-gesättigten Carbonsäure mit 2 bis 18 Kohlenstoffatomen in dem man eines 3-Alkoxy 19-nor pregna 3,5(10) 17(20) Trien II der Einwirkung eines formylierenden Reagenz unter der Bedingungen der Vilsmeier-Hack's Reaktion unterwirft, um eine 6-formylierte Verbindung der allgemeinen Formeln III

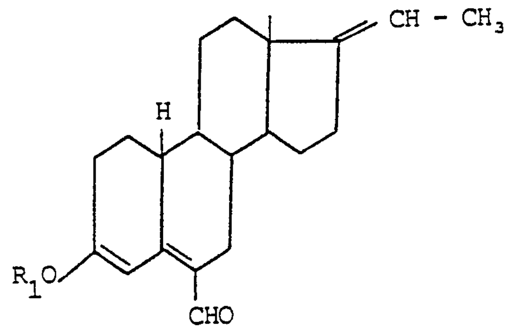

(III)

(in E oder Z Form) worin $R_1$ für einen niedriges Alkyl-Rest oder einen niedriges Cycloalkyl Rest steht zu erhalten die man mittels ein Alkalimetalmischydrid zu eine 6-hydroxymethylierte Verbindung der allgemeinen Formeln IV

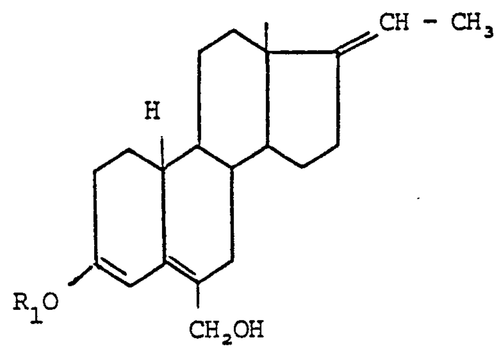

(IV)

(in E oder Z Form) worin $R_1$ dieselbe Bedeutungen als vorher, besitzt reduziert, die man die Einwirkung einer starken Saüre oder einer organischen Säure unterwirft um der 3-keto 6-methylen Derivat der allgemeinen Formeln V

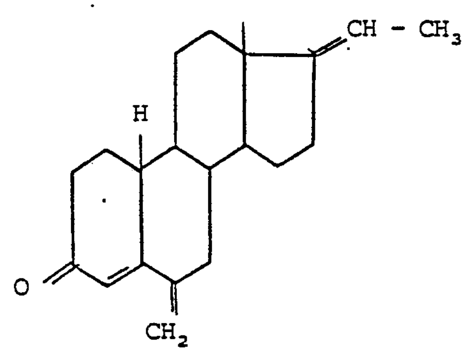

(V)

(in E oder Z Form) zu herstellen und dieses durch Einwirkung eines isomerisierenden Katalyst isomerisiert wird, um 3-Keto 6-methyl 19-nor pregna 4,6,17(20) Trien der allgemeinen Formeln VI zu herstellen dadurch gekennzeichnet dass man diese 3-Keto 19-nor Δ4,6,17(20) pregnatrienische Verbindung der allgemeinen Formeln VI

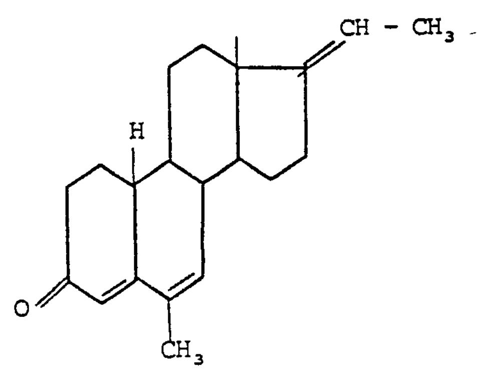

(VI)

(in E oder Z Form) der Einwirkung eines gleichzeitig verbrauchten aus einer Menge von Osmium Tetroxyd mit einer beteudende molar Menge eines Hydroperoxyd eines N-oxyd von tertiären Amin vermischt bestehenden bis-hydroxylierenden Reagenz unterwirft, um eines 17α-Hydroxy 19-nor Δ4,6-pregna dien der allgemeinen Formeln I

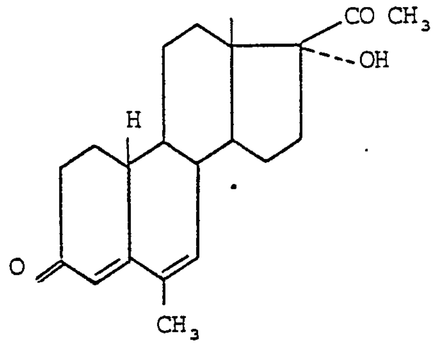

(I)

zu erhalten und dieses gewünschten-falls durch Acylierung mittels einen funktionalen Derivate einer organischen Carbonsäure in einem 17α-acylierten Derivate überführt.

2. Verfahren nach Anspruch 1 in dem die bis-hydroxylierende Umsetzung in einem inerten tertiären Alkohol in Gegenwart einem pyridische Base durchgeführt wird.

3. Als Zwischenprodukten, die 19-Nor pregna-trienen der allgemeinen Formeln III

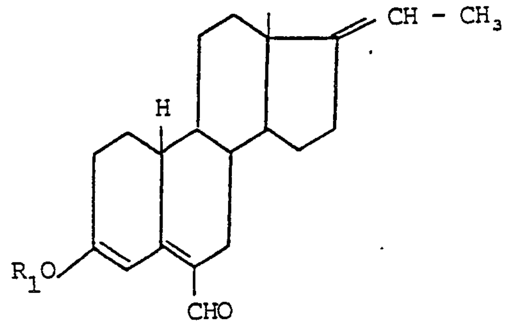

(III)

(als E oder Z Isomeren) worin $R_1$ für einen niedrigen Alkyl Rest oder ein niedrigen Cycloalkyl Rest steht.

4. Als Zwischenprodukten, die 6-Hydroxy methylierte Derivaten der allgemeinen Formeln IV

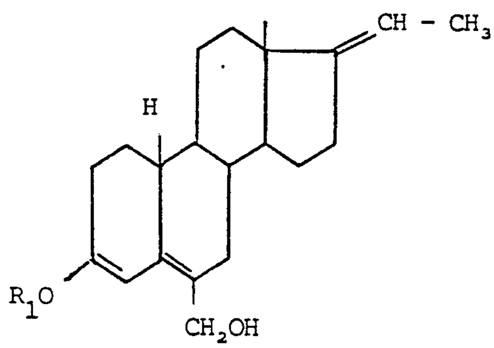

(IV)

(als E oder Z Isomeren) worin $R_1$ dieselbe Bedeutungen als vorher besitzt.

5. Als Zwischenprodukt, der 3-Keto 6-methylen Derivat der Formeln V

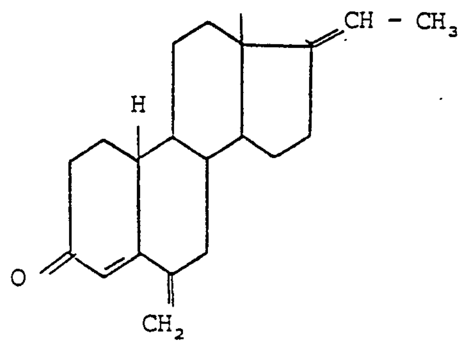

(V)

(als E oder Z Isomeren).

6. Als Zwischenprodukt, 3-Keto 19-nor pregna 4,6,17(20) Trien der allgemeinen Formeln VI

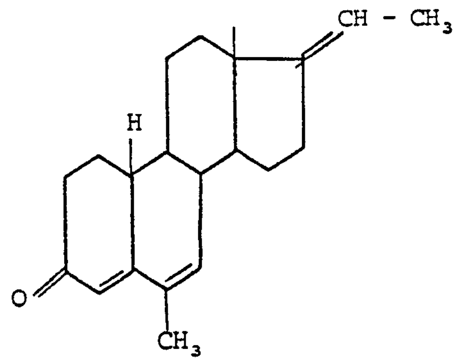

(VI)

(als E oder Z Isomeren).